# EUROPEAN PATENT APPLICATION

(11) **EP 4 693 322 A1**
(43) Date of publication of application: **11.02.2026**
(21) Application number: 24306337.7
(22) Date of filing: 08.08.2024
(51) Int. Cl.: G16H 20/17, G16H 40/40

(54) **CALIBRATION METHOD FOR DRUG DELIVERY DEVICE**

(71) Applicant: Becton Dickinson France, 38800 Le Pont-de-Claix (FR)
(72) Inventor: MCGRATH, Sean, Dublin, D05 V5W9 (IE)
(74) Representative: Germain Maureau

(57) **Abstract**

A method of calibration of a drug delivery device including a pump, a fluid line, a reservoir, and a power source, includes actuating the pump a predetermined number of cycles to move fluid through the fluid line, determining an estimated pressure value within the fluid line for each cycle of the predetermined number of cycles, determining a baseline pressure level including an average of the estimated pressure values, delivering a discrete, predetermined volume of fluid through the fluid line via the pump, determining a delivery pressure value within the fluid line, determining a difference between the delivery pressure value and the baseline pressure level, and delivering a dose volume of fluid through the fluid line via the pump at a predetermined delivery profile based on the difference between the delivery pressure value and the baseline pressure level.

## Description

### BACKGROUND OF THE INVENTION

### Field of the Invention

The present disclosure relates to a calibration method for a drug delivery device.

### Description of Related Art

Wearable medical devices, such as automatic injectors, have the benefit of providing therapy to the patient at a location remote from a clinical facility and/or while being worn discretely under the patient's clothing. The wearable medical device can be applied to the patient's skin and configured to automatically deliver a dose of a pharmaceutical composition within a predetermined time period after applying the wearable medical device to the patient's skin, such as after a 27 hour delay. After the device delivers the pharmaceutical composition to the patient, the patient may subsequently remove and dispose of the device.

In certain circumstances, due to the medium in which the liquid is being injected, the flow of fluid leaving the device may be impaired, which can lead to increased pressure in the fluid line of the device. When the pressure rises above a certain threshold, the integrity of the fluid path may be compromised causing a leak within the device and a failure to deliver the full dose of medicament. A fluid leak within the device may also cause damage to the device and subsequent system failures as well as potential contamination concerns due to contact between the fluid and the device.

Human subcutaneous tissue is composed of various cell types, extracellular matrix (ECM) constituents, microstructures, and macroscopic arrangement of cells and ECM. Those elements contribute to the mechanical properties of the tissue. The tissue may also include lymphatic system and blood vessels, and has intrinsic fluid absorption and retention properties. These characteristics vary among individuals, location within the body, and over time may cause variable degrees of resistance to the infusion of fluids at the site of injection. When the resistance of the tissue is too high or the absorption rate is too low for a given delivery flow rate from the device, the pressure may build up and reach valves above the threshold where the fluid line and other components may be compromised.

### SUMMARY OF THE INVENTION

In one aspect or embodiment, a method of calibration of a drug delivery device including a pump, a fluid line, a reservoir, and a power source, includes: a) actuating the pump a predetermined number of cycles to move fluid through the fluid line, wherein the predetermined number of cycles is greater than one; b) determining an estimated pressure value within the fluid line for each cycle of the predetermined number of cycles; c) determining a baseline pressure level including an average of the estimated pressure values; d) delivering a discrete, predetermined volume of fluid through the fluid line via the pump; e) determining a delivery pressure value within the fluid line; f) determining a difference between the delivery pressure value and the baseline pressure level; and g) delivering a dose volume of fluid through the fluid line via the pump at a predetermined delivery profile based on the difference between the delivery pressure value and the baseline pressure level.

The predetermined delivery profile may include a plurality of power levels for the pump. The plurality of power levels may include at least one of 25% of maximum pump speed, 50% of maximum pump speed, 75% of maximum pump speed, and 100% of maximum pump speed.

The method may include identifying a viscosity of the fluid based on the difference between the delivery pressure value and the baseline pressure level.

The discrete, predetermined volume of fluid may be 15-50 microliters. The dose volume of fluid may be greater than 1 milliliter.

Delivering the dose volume of fluid may include selecting the predetermined delivery profile from one of an immediate delivery of the dose volume of fluid, continuous delivery of the dose volume of fluid over a time period greater than 24 hours, and a delayed delivery of the dose volume of fluid by a time period greater than 1 hour.

Determining the estimated pressure value may include measuring a current of a motor during actuation of the pump.

One or more features of the method may be performed using a microcontroller including at least one processor.

In one aspect or embodiment, a drug delivery device includes a power source, a reservoir configured to receive a fluid, a fluid line in fluid communication with the reservoir, a pump configured to deliver the fluid from the reservoir to the fluid line, a microcontroller having at least one processor programmed or configured to cause the device to: a) actuate the pump a predetermined number of cycles to move fluid through the fluid line, wherein the predetermined number of cycles is greater than one; b) determine an estimated pressure value within the fluid line for each cycle of the predetermined number of cycles; c) determine a baseline pressure level including an average of the estimated pressure values; d) deliver a discrete, predetermined volume of fluid through the fluid line via the pump; e) determine a delivery pressure value within the fluid line; f) determine a difference between the delivery pressure value and the baseline pressure level; and g) determine a dose volume of fluid through the fluid line via the pump at a predetermined delivery profile based on the difference between the delivery pressure value and the baseline pressure level.

### BRIEF DESCRIPTION OF THE DRAWINGS

The above-mentioned and other features and advantages of this disclosure, and the manner of attaining them, will become more apparent and the disclosure itself will be better understood by reference to the following descriptions of embodiments of the disclosure taken in conjunction with the accompanying drawings, wherein:
**FIG. 1** is a perspective view of a drug delivery device according to one aspect or embodiment of the present application;
**FIG. 2** is a perspective view of the drug delivery device of FIG. 1, with a top cover removed;
**FIG. 3** is a schematic of the drug delivery device of FIG. 1;
**FIG. 4** is a graph of maximum delivery pressure versus a starting pressure; and
**FIG. 5** is a schematic of a calibration algorithm according to one aspect or embodiment of the present application.

Corresponding reference characters indicate corresponding parts throughout the several views. The exemplifications set out herein illustrate exemplary embodiments of the disclosure, and such exemplifications are not to be construed as limiting the scope of the disclosure in any manner.

### DETAILED DESCRIPTION OF THE INVENTION

Spatial or directional terms, such as "left", "right", "inner", "outer", "above", "below", and the like, are not to be considered as limiting as the invention can assume various alternative orientations.

All numbers used in the specification and claims are to be understood as being modified in all instances by the term "about". By "about" is meant a range of plus or minus ten percent of the stated value. As used in the specification and the claims, the singular form of "a", "an", and "the" include plural referents unless the context clearly dictates otherwise. The terms "first", "second", and the like are not intended to refer to any particular order or chronology, but instead refer to different conditions, properties, or elements. By "at least" is meant "greater than or equal to".

As used herein, the term "at least one of" is synonymous with "one or more of'. For example, the phrase "at least one of A, B, and C" means any one of A, B, and C, or any combination of any two or more of A, B, and C. For example, "at least one of A, B, and C" includes one or more of A alone; or one or more of B alone; or one or more of C alone; or one or more of A and one or more of B; or one or more of A and one or more of C; or one or more of B and one or more of C; or one or more of all of A, B, and C.

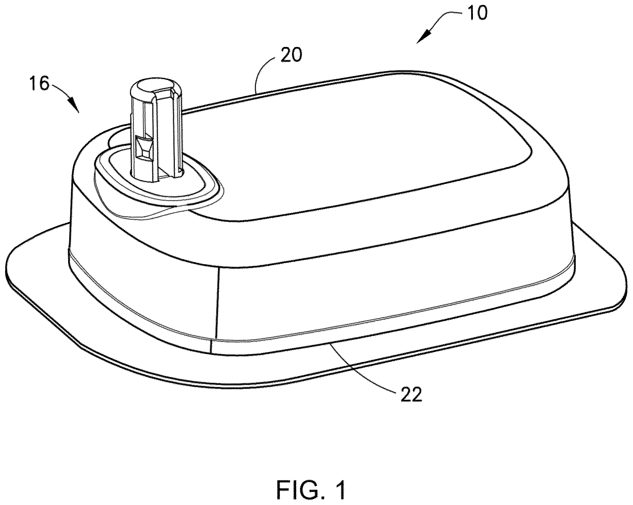

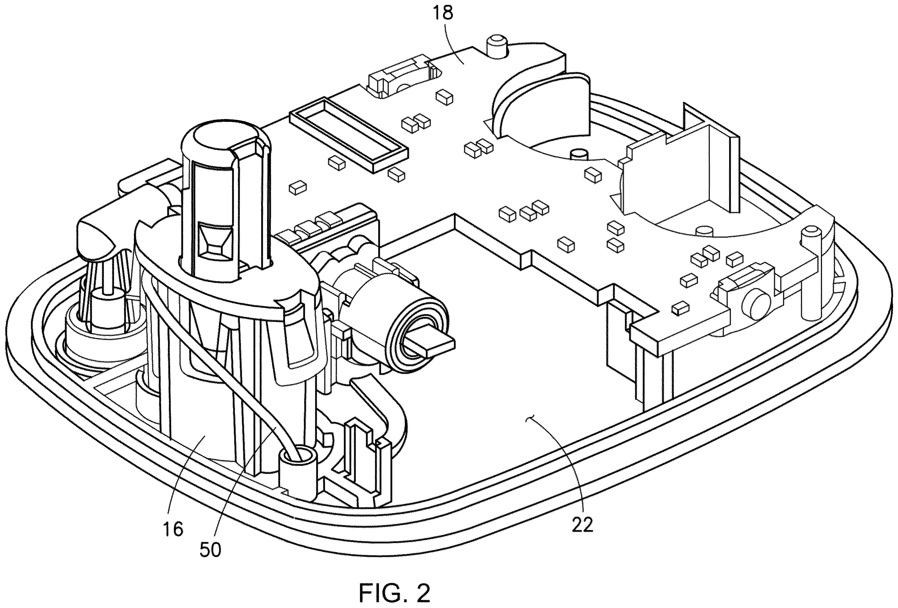

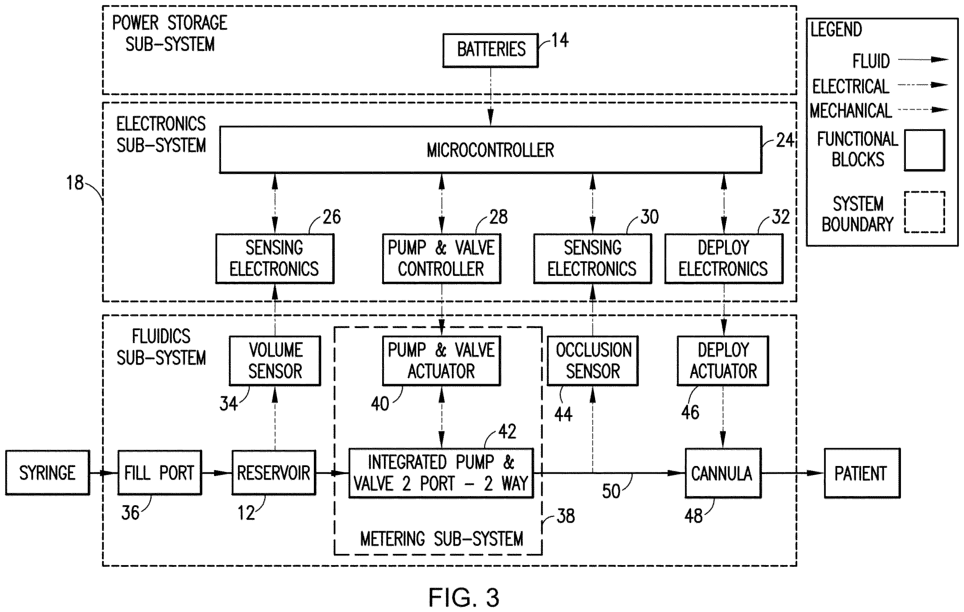

Referring to FIGS. 1-3, a drug delivery device 10 includes a reservoir 12, a power source 14, an insertion mechanism 16, control electronics 18, a cover 20, and a base 22. In one aspect or embodiment, the drug delivery device 10 is a wearable automatic injector, such as an insulin or bone marrow stimulant delivery device. The drug delivery device 10 may be mounted onto the skin of a patient and triggered to inject a pharmaceutical composition from the reservoir 12 into the patient. The drug delivery device 10 may be pre-filled with the pharmaceutical composition, or it may be filled with the pharmaceutical composition by the patient or medical professional prior to use.

The drug delivery device 10 is configured to deliver a dose of a pharmaceutical composition, e.g., any desired medicament, into the patient's body by a subcutaneous injection at a slow, controlled injection rate. Exemplary time durations for the delivery achieved by the drug delivery device 10 may range from about 5 minutes to about 60 minutes, but are not limited to this exemplary range. Exemplary volumes of the pharmaceutical composition delivered by the drug delivery device 10 may range from about 0.1 milliliters to about 10 milliliters, but are not limited to this exemplary range. The volume of the pharmaceutical composition delivered to the patient may be adjusted.

Referring again to FIGS. 1-3, in one aspect or embodiment, the power source 14 is a DC power source including one or more batteries. The control electronics 18 include a microcontroller 24, sensing electronics 26, a pump and valve controller 28, sensing electronics 30, and deployments electronics 32, which control the actuation of the drug delivery device 10. The drug delivery device 10 includes a fluidics sub-system that includes the reservoir 12, a volume sensor 34 for the reservoir 12, a reservoir fill port 36, and a metering system 38 including a pump and valve actuator 40 and a pump and valve mechanism 42. The fluidic sub-system may further include an occlusion sensor 44, a deploy actuator 46, a cannula 48 for insertion into a patient's skin, and a fluid line 50 in fluid communication with the reservoir 12 and the cannula 48. In one aspect or embodiment, the insertion mechanism 16 is configured to move the cannula 48 from a retracted position positioned entirely within the device 10 to an extended position where the cannula 48 extends outside of the device 10. The drug delivery device 10 may operate in the same manner as discussed in U.S. Patent No. 10,449,292 to Pizzochero et al, incorporated herein by reference.

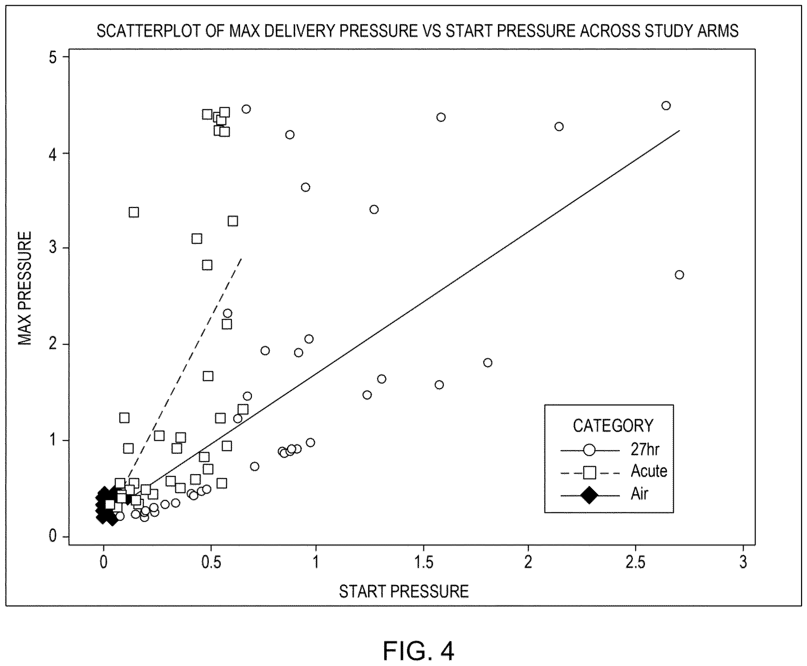

Referring to FIG. 4, devices with a high starting pressure generally tend to have a higher maximum delivery pressure over the entire delivery of fluid. If a high starting pressure is measured directly or indirectly (*i.e.,* via current draw), an infusion or injection rate can be adjusted within the device to avoid the device generating high internal pressures that may trigger an occlusion alarm. Similarly, this can be done to detect the viscosity of fluid that the device is either injecting/infusing. As viscosity of a drug increases, the more resistance/pressure is needed to push the fluid from the device.

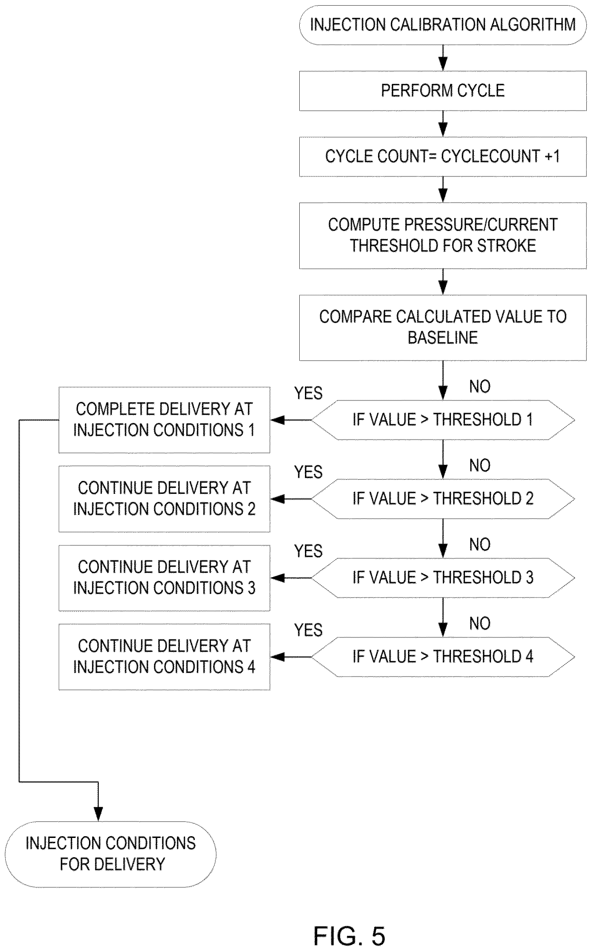

Referring to FIGS. 1-5, in one aspect or embodiment, a method of calibration of the drug delivery device 10 includes: a) actuating the pump 42 a predetermined number of cycles to move fluid through the fluid line, wherein the predetermined number of cycles is greater than one; b) determining an estimated pressure value within the fluid line 50 for each cycle of the predetermined number of cycles; c) determining a baseline pressure level including an average of the estimated pressure values; d) delivering a discrete, predetermined volume of fluid through the fluid line via the pump 42; e) determining a delivery pressure value within the fluid line 50; f) determining a difference between the delivery pressure value and the baseline pressure level; and g) delivering a dose volume of fluid through the fluid line via the pump 42 at a predetermined delivery profile based on the difference between the delivery pressure value and the baseline pressure level. In one aspect or embodiment, the predetermined number of cycles is three or more pump cycles.

The predetermined delivery profile may include a plurality of power levels for the pump 42. The plurality of power levels may include at least one of 25% of maximum pump speed, 50% of maximum pump speed, 75% of maximum pump speed, and 100% of maximum pump speed, which correspond to different flow rates provided by the pump 42.

In one aspect tor embodiment, the method includes identifying a viscosity of the fluid based on the difference between the delivery pressure value and the baseline pressure level. Known fluid or medication have a viscosity range and higher viscosity fluids will have a greater difference between the delivery pressure value and the baseline pressure level. Based on testing or other known values, the difference between the delivery pressure value and the baseline pressure level can be correlated with viscosity, which can then be used to determine the fluid being delivered and a suitable delivery profile may be selected based on the fluid. Further, in some aspects or embodiments, the method includes identifying a fluidic resistance of a medium that is being injection or infused based on a difference between the delivery pressure value and the baseline pressure level.

In one aspect or embodiment, the discrete, predetermined volume of fluid may be 5-20 microliters and the dose volume of fluid may be greater than 1 milliliter. In one aspect or embodiment, the dose volume of fluid is 0.1 milliliter to 3 milliliters.

In one aspect or embodiment, delivering the dose volume of fluid may include selecting the predetermined delivery profile from one of an immediate delivery of the dose volume of fluid, continuous delivery of the dose volume of fluid over a time period greater than 24 hours, and a delayed delivery of the dose volume of fluid by a time period greater than 1 hour. The continuous delivery may be a singular pulse of drug every minute for up to 3 days or multiple pulses of drug at once every minute for up to 3 days. In one aspect or embodiment, the continuous delivery may be a singular pulse of drug every five minutes for up to 3 days or multiple pulses of drug at once every five minutes for up to 3 days. In one aspect or embodiment, the continuous delivery may be a singular pulse of drug every ten minutes for up to 3 days or multiple pulses of drug at once every ten minutes for up to 3 days.

In one aspect or embodiment, the baseline pressure level represents a minimum level to operate the pump 42, *i.e.*, when the pump 42 is not blocked or occluded, such that any increase above the baseline pressure level can be attributed to the pump 42 having to operate with at least a partially occluded fluid path. In one aspect or embodiment, the pressure within the fluid line 50 is determined by measuring a current of the drug delivery device 10 during actuation of the pump 42. The measuring of the current of the drug delivery device 10 may include subtracting a reference or baseline current value from a peak current value during an actuation cycle of the pump 42 to determine a stroke current value, although other suitable current detection arrangements may be utilized. The stroke current value is utilized to estimate the downstream pressure of the fluid line 50 for the particular actuation cycle of the pump 42. For example, the stroke current value can be corresponded to various downstream pressure levels through testing or benchmarking such that the stroke current value can be used to estimate the pressure level of the fluid line 50.

Although the invention has been described in detail for the purpose of illustration based on what is currently considered to be the most practical and preferred embodiments, it is to be understood that such detail is solely for that purpose and that the invention is not limited to the disclosed embodiments, but, on the contrary, is intended to cover modifications and equivalent arrangements that are within the spirit and scope of the appended claims. For example, it is to be understood that the present invention contemplates that, to the extent possible, one or more features of any embodiment can be combined with one or more features of any other embodiment.

## Claims

1. A method of calibration of a drug delivery device comprising a pump, a fluid line, a reservoir, and a power source, the method comprising:
a) actuating the pump a predetermined number of cycles to move fluid through the fluid line, wherein the predetermined number of cycles is greater than one;
b) determining an estimated pressure value within the fluid line for each cycle of the predetermined number of cycles;
c) determining a baseline pressure level comprising an average of the estimated pressure values;
d) delivering a discrete, predetermined volume of fluid through the fluid line via the pump;
e) determining a delivery pressure value within the fluid line;
f) determining a difference between the delivery pressure value and the baseline pressure level; and
g) delivering a dose volume of fluid through the fluid line via the pump at a predetermined delivery profile based on the difference between the delivery pressure value and the baseline pressure level.

2. The method of claim 1, wherein the predetermined delivery profile comprises a plurality of power levels for the pump.

3. The method of claim 1, wherein the plurality of power levels comprises at least one of 25% of maximum pump speed, 50% of maximum pump speed, 75% of maximum pump speed, and 100% of maximum pump speed.

4. The method of claim 1, further comprising:
identifying a viscosity of the fluid based on the difference between the delivery pressure value and the baseline pressure level.

5. The method of claim 1, wherein the discrete, predetermined volume of fluid is 5-20 microliters.

6. The method of claim 1, wherein the dose volume of fluid is greater than 1 milliliter

7. The method of claim 1, wherein delivering the dose volume of fluid comprises:
selecting the predetermined delivery profile from one of an immediate delivery of the dose volume of fluid, continuous delivery of the dose volume of fluid over a time period greater than 24 hours, and a delayed delivery of the dose volume of fluid by a time period greater than 1 hour.

8. The method of claim 1, wherein determining the estimated pressure value comprises measuring a current of a motor during actuation of the pump.

9. The method of any of claims 1-8, wherein a) through g) are performed using a microcontroller comprising at least one processor.

10. A drug delivery device comprising:
a power source;
a reservoir configured to receive a fluid;
a fluid line in fluid communication with the reservoir;
a pump configured to deliver the fluid from the reservoir to the fluid line; and
a microcontroller comprising at least one processor programmed or configured to cause the device to:
a) actuate the pump a predetermined number of cycles to move fluid through the fluid line, wherein the predetermined number of cycles is greater than one;
b) determine an estimated pressure value within the fluid line for each cycle of the predetermined number of cycles;
c) determine a baseline pressure level comprising an average of the estimated pressure values;
d) deliver a discrete, predetermined volume of fluid through the fluid line via the pump;
e) determine a delivery pressure value within the fluid line;
f) determine a difference between the delivery pressure value and the baseline pressure level; and
g) determine a dose volume of fluid through the fluid line via the pump at a predetermined delivery profile based on the difference between the delivery pressure value and the baseline pressure level.
